Europäisches Patentamt

European Patent Office

Office européen des brevets

(19)

(11) Publication number: **0 402 874**
**A2**

## (12) EUROPEAN PATENT APPLICATION

(21) Application number: **90111127.8**

(22) Date of filing: **12.06.90**

(51) Int. Cl.5: **C07C 19/08, C07C 17/10**

(30) Priority: **13.06.89 IT 2085189**

(43) Date of publication of application:
**19.12.90 Bulletin 90/51**

(84) Designated Contracting States:
**BE CH DE ES FR GB LI NL SE**

(71) Applicant: **AUSIMONT S.r.l.**
**31, Foro Buonaparte**
**I-20100 Milano(IT)**

(72) Inventor: **Gervasutti, Claudio, Dr.**
**2/4, Via P. Sarpi**
**I-30172 Mestre, Venezia(IT)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) **Process for preparing 1,1,1-trifluoro-2,2-dichloroethane.**

(57) 1,1,1-Trifluoro-2,2-dichloroethane is prepared by reacting, in the gas phase, 1,1,1-trifluoro-2-chloroethane with chlorine. The formation of the by-product 1,1,1-trifluorotrichloroethane is prevented by effecting the reaction with a chlorine/organic compound molar ratio of not higher than 1 at a temperature of from $350\,°C$ to $450\,°C$ and with residence times of the reagents in the reactor of from 5 to 25 seconds.

EP 0 402 874 A2

## PROCESS FOR PREPARING 1,1,1-TRIFLUORO-2,2-DICHLOROETHANE

The present invention relates to a process for (continuously) preparing 1,1,1-trifluoro-2,2-dichloroethane by reaction between gaseous 1,1,1-trifluoro-2-chloroethane and chlorine, which allows to obtain the product in a yield of about 100%, free of the by-product 1,1,1-trifluoro-trichloroethane.

1,1,1-Trifluoro-2,2-dichloroethane is a well-known chlorofluorohydrocarbon (HCFC 123) now internationally recognized as the most likely substitute for CFC 11 in the fields of aerosols, refrigeration and foamed articles, due to its ability of decomposing in the atmosphere and therefore of not chemically interacting with the tropospheric ozone layer.

Several processes for preparing 1,1,1-trifluoro-2,2-dichloroethane by reaction of the corresponding monochlorinated compound with chlorine are known in the art.

According to US-A-4,145,368, the two components are reacted under formation of a mixture consisting of 1,1,1-trifluoro-2,2-dichloroethane, 1,1,1-trifluorotrichloroethane and unreacted material. Thereafter the dichlorinated product is recovered and the trichlorinated derivative subsequently is reacted with additional amounts of the starting monochlorinated derivative, in the presence of catalysts consisting of metal oxides or oxyfluorides or of activted carbon, in order to obtain further quantities of 1,1,1-trifluoro-2,2-dichloroethane by means of a "coproportionation" reaction between the two reagents.

The latter reaction affords yields of not higher than 15%. According to US-A-4,060,469, 1,1,1-trifluoro-2,2-dichloroethane is prepared by reacting the monochlorinated· derivative with chlorine, without formation of the perchlorinated by- product $CF_3CCl_3$. operating under photochemical chlorination conditions with less than equimolar amounts of chlorine.

However, said process cannot be employed on an industrial scale because of the difficulty of constructing photochemical reactors of suitable dimensions.

Finally, according to US-A-2,644,845, 1,1,1-trifluoro-2,2-dichloroethane is prepared, in admixture with considerable amounts of perchlorinated product ($CF_3CCl_3$) and monochlorinated product, by means of thermal chlorination of 1,1,1-trihaloalkanes with chlorine, carried out at temperatures higher than 200°C and generally ranging from 350°C to 450°C. Said patent points out the difficulty of obtaining 1,1,1-trifluoro-2,2-dichloroethane from $CF_3CH_2Cl$ without concomitant and significant formation of perhalogenated compound $CF_3CCl_3$, which is favoured by a higher reaction rate.

It is, therefore, apparent that there is still a need for a simple industrial process by which it is possible to (continuously) prepare large amounts of 1,1,1-trifluoro-2,2-dichloroethane with a minimum production of perhalogenated by-product 1,1,1-trifluorotrichloroethane ($CF_3CCl_3$).

It has now surprisingly been found that by using a particular combination of reaction temperature, residence time of the reagents in the reactor and molar ratio of the reagents it is possible to obtain 1,1,1-trifluoro-2,2-dichloroethane substantially free of 1,1,1-trifluorotrichloroethane.

In particular, the operative conditions which permit to obtain the desired result comprise reacting, in the gas phase, chlorine with 1,1,1-trifluoro-2-chloroethane in molar ratios of the former to the latter not exceeding 1, at temperatures ranging from 350°C to 450°C and with both reagents remaining under the above reaction conditions for intervals ranging from 5 to 25 seconds.

Thus, the present invention provides a process for preparing 1,1,1-trifluoro-2,2-dichloroethane which comprises reacting, in the gase phase and at a temperature ranging from 350°C to 450°C, 1,1,1-trifluoro-2-chloroethane with chlorine in a molar ratio chlorine/l,1,1-trifluoro-2-chloroethane of not higher than 1 and with contact times, or periods of times for which the reagents remain under the above reaction conditions, ranging from 5 to 25 seconds.

Preferably chlorine/1,1,1-trifluoro-2-chloroethane molar ratios ranging from 0.3 to 1, particularly from 0.5 to 0.8, are employed. With molar ratios lower than 0.3, a significant decrease in the conversion occurs, and not even an increase of the reaction temperature and of the contact time to the maximum allowable levels can compensate said effect.

Chlorine may be used in the pure state or diluted with an inert gas, for example nitrogen, helium or argon.

Preferred reaction temperatures range from 350°C to 400°C.

Preferred values of the time during which the reagents remain under the above reaction conditions range from 10 to 15 seconds. The reaction is conducted in the gas phase, preferably at atmospheric pressure. Preferably tubular reactors made of a metal material such as nickel and iron, alloys such as Inconel, Hastelloy, or stainless steel are employed. Stainless steel is the most preferred material.

Besides the substantial absence of 1,1,1-trifluorotrichloroethane in the reaction mixture, the process of the present invention offers the further advantage, as compared to the processes of the prior art, of being a non-catalytic process (i.e. a process free from the drawbacks stemming from catalyst exhaustion or decay) and of being carried out in a single reaction stage.

According to a preferred embodiment of the present invention, the reagents are continuously fed to the reactor, which is thermoregulated at the desired temperature, whereafter the vapors leaving the reactor are subjected to scrubbing with an aqueous solution at 5-20% by weight of an alkali hydroxide. Thereafter they are dried, for example over CaCl$_2$, and cooled in a dry ice trap until complete condensation is achieved.

Unreacted 1,1,1-trifluoro-2-chloroethane can be recovered from the reaction mixture by means of distillation and then, after addition of chlorine in the amounts indicated hereinbefore, it can be recycled to the reactor for further conversion to 1,1,1-trifluoro-2,2-dichloroethane.

In order to promote the mixing between the reagents and to secure a homogeneous temperature throughout the entire gaseous mixture, the reactor generally is filled with an inert material in the form of shavings or the like, having irregular shapes and sizes, however, in amounts such as to not cause too high a pressure drop in the gaseous stream flowing through the reactor.

The following examples are given to better illustrate the present invention, without being, however, a limitation thereof.

EXAMPLE 1

A total of 0.86 moles/h of a mixture, preheated to 200°C, composed of anhydrous chlorine, 1,1,1-trifluoro-2-chloroethane and nitrogen in molar ratios chlorine/trifluoro-2-chloroethane/nitrogen = 0.8:1:1.13, was introduced,at atmospheric pressure, into a cylindrical AISI 316 steel reactor having an internal diameter of 20 mm and a length of 500 mm (reactor volume = 157 ml), filled with AISI 316 steel shavings up to a "usable" volume of 120 ml, and thermoregulated at 380°C.

The residence time of the vapors forming the feed mixture in the reactor was 10 seconds. The vapors leaving the reactor were scrubbed and condensed in an aqueous solution of NaOH at 10% in order to remove the hydrochloric acid which had formed, as well as the unreacted chlorine, if any.

The recovered organic product, subjected to gas-chromatographic analysis, exhibited the following composition:
1,1,1-trifluoro-2,2-dichloroethane 21% by weight
1,1,1-trifluorotrichloroethane absent
1,1,1-trifluoro-2-chloroethane 79%.

EXAMPLE 2

A total of 0.78 moles/h of a mixture, preheated to 200°C, consisting of chlorine, 1,1,1-trifluoro-2-chloroethane and nitrogen in molar ratios chlorine/organic product/nitrogen = 0.5:1.0:1.13, was introduced, at atmospheric pressure, into the reactor of example 1, thermoregulated at 400°C.

The residence time of the vapors forming the feed mixture in the reactor was 10 seconds. By following the procedure of example 1 an organic mixture was recovered which, when examined by gas-chromatography, exhibited the following composition:
1,1,1-trifluoro-2,2-dichloroethane 15.0% by weight
1,1,1-trifluorotrichloroethane 1.0% by weight
unreacted 1,1,1-trifluoro-2-chloroethane 84.0% by weight.

Claims

1. Process for preparing 1,1,1-trifluoro-2,2-dichloroethane which comprises reacting, in the gas phase and at a temperature of from 350°C to 450°C, 1,1,1-trifluoro-2-chloroethane with chlorine in a molar ratio chlorine/1,1,1-trifluoro-2-chloroethane of not higher than 1 and with contact times of from 5 to 25 seconds.

2. Process according to claim 1, wherein the chlorine/1,1,1-trifluoro-2-chloroethane molar ratio ranges from 0.5 to 0.8.

3. Process according to any one of claims 1 and 2, wherein the contact times range from 10 to 15 seconds.

4. Process according to any one of the preceding claims, wherein the reaction temperature ranges from 350°C to 400°C.

5. Process according to any one of claims 1 to 4, which is conducted continuously.